(19) Europäisches Patentamt
European Patent Office
Office européen des brevets

(11) Publication number: 0 371 388 A2

(12) EUROPEAN PATENT APPLICATION

(21) Application number: 89121580.8

(22) Date of filing: 22.11.89

(51) Int. Cl.[5]: C07D 219/10, C07D 221/22, A61K 31/435, C07C 255/58

(30) Priority: 28.11.88 GB 8827704

(43) Date of publication of application: 06.06.90 Bulletin 90/23

(84) Designated Contracting States: AT BE CH DE ES FR GB GR IT LI LU NL SE

(71) Applicant: FUJISAWA PHARMACEUTICAL CO., LTD.
4-7, Doshomachi 3-chome Chuo-ku
Osaka-shi Osaka 541(JP)

(72) Inventor: Takasugi, Hisashi
14-33, Hamaguchinishi 1-chome Suminoe-ku
Osaka-shi Osaka 559(JP)
Inventor: Kuno, Atsushi
24-6, Shinkofudai 5-chome Toyono-cho
Toyono-gun Osaka 563-01(JP)
Inventor: Ohkubo, Mitsuru
1-65, Fushimidai 5-chome Inagawa-cho
Kawabe-gun Hyogo 666-02(JP)
Inventor: Sakai, Hiroyoshi
28-134, Kowada-Hirao
Uji-shi Kyoto 611(JP)

(74) Representative: Türk, Gille, Hrabal
Brucknerstrasse 20
D-4000 Düsseldorf 13(DE)

(54) **Acridine derivatives.**

(57) A compound of the formula :

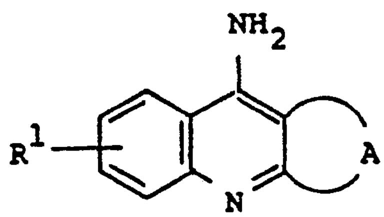

wherein $R^1$ is hydrogen, halogen, hydroxy, lower alkoxy, lower alkyl or mono(or di or tri)halo(lower)alkyl, and

EP 0 371 388 A2

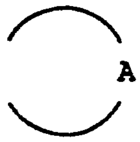 is 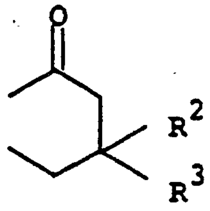 , 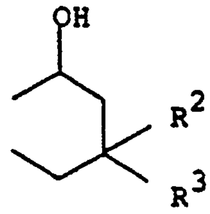 , 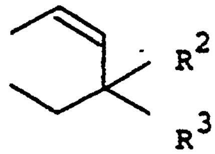 ,

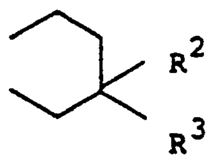 , 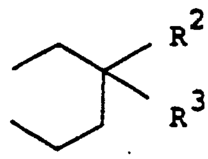 or 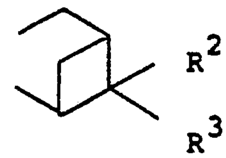

(in which $R^2$ and $R^3$ are each lower alkyl),
and a pharmaceutically acceptable salt thereof, processes for their preparation and pharmaceutical compositions comprising them as an active ingredient in admixture with pharmaceutically acceptable carriers. The invention also relates to intermediates of the formula

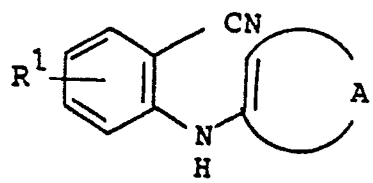

# ACRIDINE DERIVATIVES

This invention relates to new acridine derivatives. More particularly, this invention relates to new acridine derivatives and pharmaceutically acceptable salts thereof, which have an acetylcholinesterase-inhibitory activity, to processes for their production, to a pharmaceutical composition containing the same and to a use thereof for manufacture of medicaments.

Accordingly, one object of this invention is to provide new and useful acridine derivatives and pharmaceutically acceptable salts thereof.

Another object of this invention is to provide processes for production of the acridine derivatives and salts thereof.

A further object of this invention is to provide a pharmaceutical composition containing, as an active ingredient, said acridine derivatives or pharmaceutically acceptable salts thereof.

Still further object of this invention is to provide a use of the acridine derivatives and pharmaceutically acceptable salts thereof for manufacture of medicaments for the therapeutic treatment of disorders in the central nervous system such as amnesia, dementia or the like.

The new acridine derivatives of this invention can be represented by the following general formula (I) :

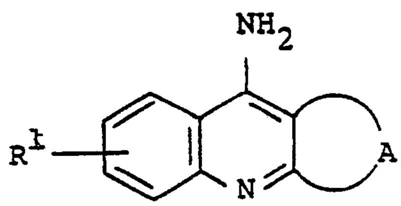

(I)

wherein $R^1$ is hydrogen, halogen, hydroxy, lower alkoxy, lower alkyl or mono(or di or tri)halo(lower)alkyl, and

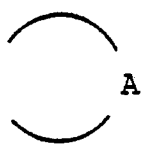 is 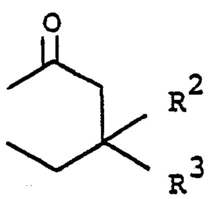 , 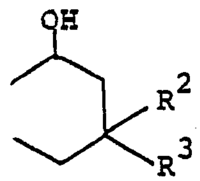 , 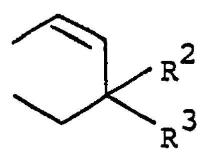 ,

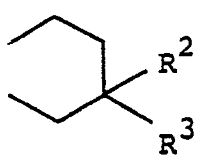 , 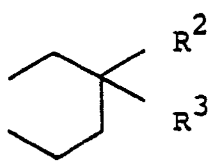 or 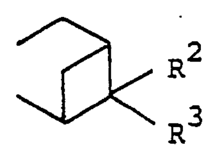

(in which $R^2$ and $R^3$ are each lower alkyl).

According to this invention, the object compound (I) and salts thereof can be prepared by the processes as illustrated in the following.

Process 1

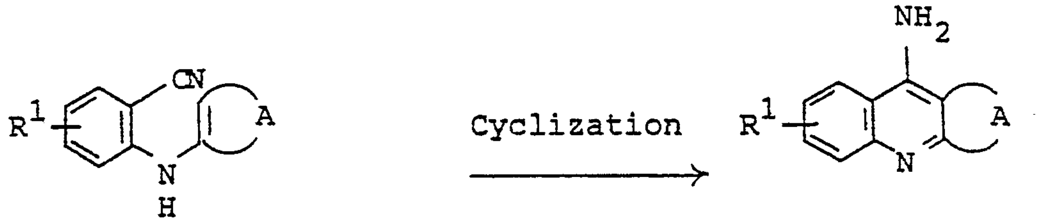


(II) or a salt thereof

(I) or a salt thereof

Process 2

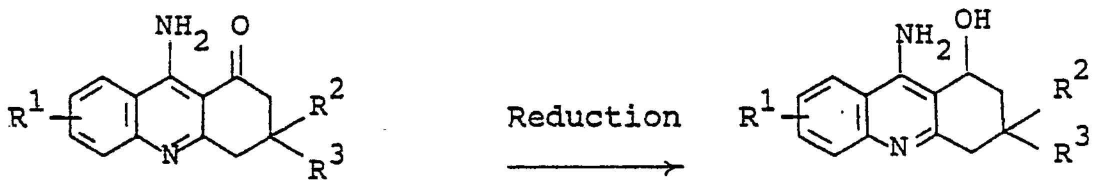


(Ia) or a salt thereof

(Ib) or a salt thereof

Process 3

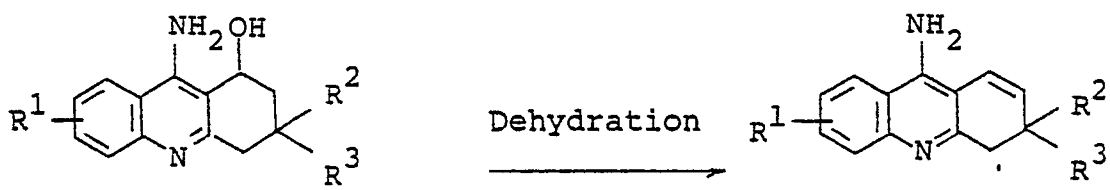


(Ib) or a salt thereof

(Ic) or a salt thereof

Process 4

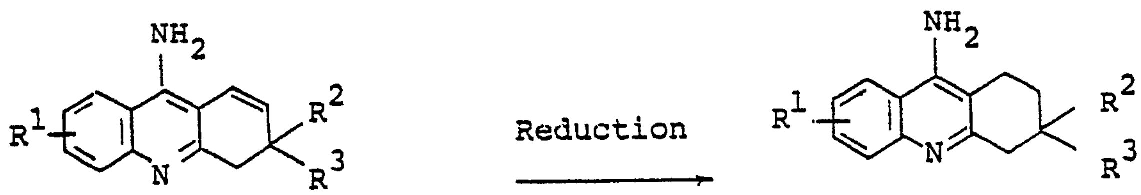


(Ic)
or a salt thereof

(Id)
or a salt thereof

Process 5

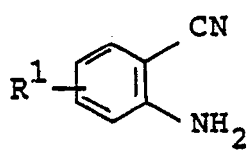

(III)
or a salt thereof

+

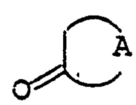

(IV)
or a salt thereof

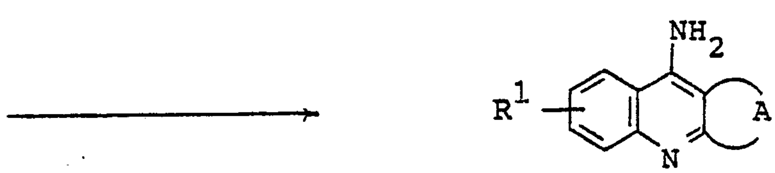

(I)
or a salt thereof

Process 6

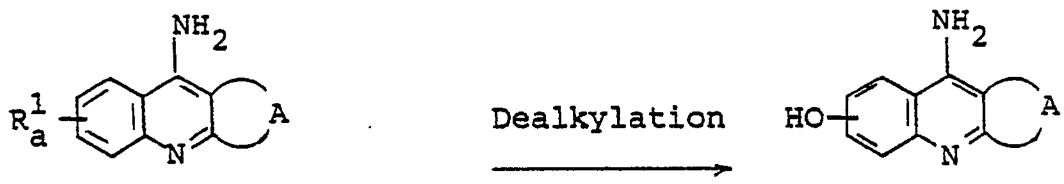


(Ie)
or a salt thereof

(If)
or a salt thereof

wherein $R^1$, $R^2$, $R^3$ and

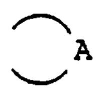

are each as defined above,
$R_a^1$ is lower alkoxy.

The starting compound (II) is novel and can be prepared by the following process.

Process A

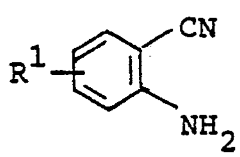

+

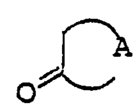

(III)
or a salt thereof

(IV)
or a salt thereof

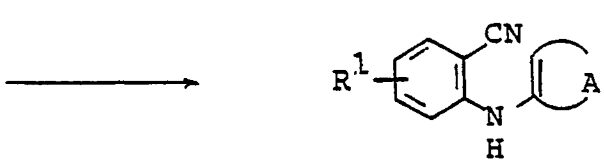

(II)
or a salt thereof

wherein $R^1$ and A are each as defined above.

In the above and subsequent descriptions of this specification, suitable examples of the various

definitions are explained in detail as follows.

The term "lower" is intended to mean 1 to 6 carbon atom(s), unless otherwise indicated.

Suitable "lower alkyl" and "lower alkyl moiety" in the term "mono(or di or tri)halo(lower)alkyl" may include a straight or branched one such as methyl, ethyl, propyl, isopropyl, butyl, isobutyl, tert-butyl, pentyl, hexyl, or the like, in which the preferred one may be $C_1$-$C_4$alkyl.

Suitable "halogen" and "halogen moiety" in the term "mono(or di or tri)halo(lower)alkyl" may include chlorine, bromine, iodine and fluorine.

Suitable "lower alkoxy" may include methoxy, ethoxy, propoxy, isopropoxy, butoxy, isobutoxy, tert-butoxy, pentyloxy, hexyloxy and the like, in which the preferred one may be $C_1$-$C_4$ alkoxy.

Suitable pharmaceutically acceptable salts of the object compound (I) are conventional non-toxic salts and may include a metal salt such as an alkali metal salt [e.g. sodium salt, potassium salt, etc.] and an alkaline earth metal salt [e.g. calcium salt, magnesium salt, etc.], an ammonium salt, an organic base salt [e.g. trimethylamine salt, triethylamine salt, pyridine salt, picoline salt, dicyclohexylamine salt, N,N′-dibenzylethylenediamine salt, etc.], an organic acid salt [e.g. formate, acetate, trifluoroacetate, maleate, tartrate, citrate, methanesulfonate, benzenesulfonate, toluenesulfonate, etc.], an inorganic acid salt [e.g. hydrochloride, hydrobromide, sulfate, phosphate, etc.], a salt with an amino acid [e.g. arginine salt, aspartic acid salt, glutamic acid salt, etc.], and the like.

The processes for production of the compound (I) of this invention are explained in detail in the following.

Process 1

The compound (I) or a salt thereof can be prepared by subjecting the compound (II) or a salt thereof to cyclization reaction. This reaction is usually carried out in the presence of an organic or inorganic base such as alkali metal (e.g. lithium, sodium, potassium, etc.), alkaline earth metal (e.g. calcium, etc.), alkali metal hydride (e.g. sodium hydride, etc.), alkaline earth metal hydride (e.g. calcium hydride, etc.), alkali metal hydroxide (e.g. sodium hydroxide, potassium hydroxide, etc.), alkali metal carbonate (e.g. sodium carbonate, potassium carbonate, etc.), alkali metal bicarbonate (e.g. sodium bicarbonate, potassium bicarbonate, etc.), alkali metal alkoxide (e.g. sodium methoxide, sodium ethoxide, potassium tert-butoxide, etc.), alkali metal alkanoic acid (e.g. sodium acetate, etc.), trialkylamine (e.g. triethylamine, etc.), pyridine compound (e.g. pyridine, lutidine, picoline, 4-dimethylaminopyridine, etc.) or the like and a metallic halide (e.g., copper chloride, copper bromide, copper iodide, etc.), or in the presence of a Lewis acid as described in process 5.

This reaction is usually carried out in a solvent such as methylene chloride, diethyl ether, dioxane, tetrahydrofuran, N,N-dimethylformamide, a mixture thereof or any other solvent which does not adversely influence the reaction.

The reaction temperature is not critical and the reaction is usually carried out at room temperature, under warming or under heating.

Process 2

The compound (Ib) or a salt thereof can be prepared by subjecting the compound (Ia) or a salt thereof to reduction reaction.

The present reduction is conducted by a conventional method such as a method of using an alkali metal borohydride (e.g., sodium borohydride, potassium borohydride, etc.), alkali metal aluminum hydride (e.g., lithium aluminum hydride, sodium aluminum hydride, etc.) or the like.

This reaction is usually carried out in a solvent such as methylene chloride, methanol, ethanol, propanol, diethyl ether, dioxane, tetrahydrofuran, N,N-dimethylformamide, a mixture thereof or any other solvent which does not adversely influence the reaction.

The reaction temperature is not critical and the reaction is usually carried out under from cooling to heating.

Process 3

The compound (Ic) or a salt thereof can be prepared by subjecting the compound (Ib) or a salt thereof

to dehydration reaction. The present dehydration is preferably conducted in the presence of the dehydrating agent such as phosphoryl chloride, phosphorus pentoxide, phosphorus pentachloride, phosphorus pentabromide, polyphosphate ester, polyphosphoric acid or the like.

This reaction is usually carried out in a solvent such as methylene chloride, tetrahydrofuran, N,N-dimethylformamide, a mixture thereof or any other solvent which does not adversely influence the reaction.

In case that the above-mentioned dehydrating agents are in liquid, they can also be used as a solvent.

The reaction temperature is not critical and the reaction is preferably carried out under warming to heating.

Process 4

The compound (Id) or a salt thereof can be prepared by subjecting the compound (Ic) or a salt thereof to reduction reaction.

Reduction is carried out in a conventional manner, including chemical reduction and catalytic reduction.

Suitable reducing agents to be used in chemical reduction are a combination of a metal (e.g. tin, zinc, iron, etc.) or metallic compound (e.g. chromium chloride, chromium acetate, etc.) and an organic or inorganic acid (e.g. formic acid, acetic acid, propionic acid, trifluoroacetic acid, p-toluenesulfonic acid, hydrochloric acid, hydrobromic acid, etc.).

Suitable catalysts to be used in catalytic reduction are conventional ones such as platinum catalysts (e.g. platinum plate, spongy platinum, platinum black, colloidal platinum, platinum oxide, platinum wire, etc.), palladium catalysts (e.g. spongy palladium, palladium black, palladium oxide, palladium on carbon, colloidal palladium, palladium on barium sulfate, palladium on barium carbonate, etc.), nickel catalysts (e.g. reduced nickel, nickel oxide, Raney nickel, etc.), cobalt catalysts (e.g. reduced cobalt, Raney cobalt, etc.), iron catalysts (e.g. reduced iron, Raney iron, etc.), copper catalysts (e.g. reduced copper, Raney copper, Ullman copper, etc.) and the like. The reduction is usually carried out in a conventional solvent which does not adversely influence the reaction such as water, methanol, ethanol, propanol, N,N-dimethylformamide, or a mixture thereof. Additionally, in case that the above-mentioned acids to be used in chemical reduction are in liquid, they can also be used as a solvent.

The reaction temperature of this reduction is not critical and the reaction is usually carried out under cooling to warming.

Process 5

The compound (I) or a salt thereof can be prepared by reacting the compound (III) or a salt thereof with the compound (IV) or a salt thereof. This reaction is usually carried out in the presence of a Lewis acid such as aluminum halide (e.g. aluminum chloride, aluminum bromide, etc.), zinc halide (e.g., zinc chloride, zinc bromide, zinc iodide, etc.), titanium (IV) halide (e.g., titanium (IV) chloride, titanium (IV) bromide, etc.), tin(IV) halide (e.g., tin (IV) chloride, tin (IV) bromide, etc.) or the like.

This reaction is usually carried out in a solvent such as dioxan, ethylene chloride, methylene chloride, tetrahydrofuran, N,N-dimethylformamide, a mixture thereof or any other solvent which does not adversely influence the reaction.

The reaction temperature is not critical and the reaction is usually carried out under warming to heating.

Process 6

The compound (If) or a salt thereof can be prepared by subjecting the compound (Ie) or a salt thereof to dealkylation reaction.

The present dealkylation is conduced by a method of using an acid such as hydrogen halide (e.g., hydrogen bromide, hydrogen iodide, etc.), Lewis acid [e.g., aluminum halide (e.g., aluminum chloride, aluminum bromide, etc.), boron trihalide (e.g., boron tribromide, etc.), etc.] or the like.

This reaction is usually carried out in a solvent such as water, methylene chloride, dioxane, tetrahydrofuran, N,N-dimethylformamide, a mixture thereof or any other solvent which does not adversely influence the reaction.

The reaction temperature is not critical and the reaction is usually carried out under heating.

Process for the preparation of the starting compound (II) is explained in the following.

Process A

The compound (II) or a salt thereof can be prepared by reacting the compound (III) or a salt thereof with the compound (IV) or a salt thereof. This reaction is preferably carried out in the presence of an acid such as an organic acid [e.g. formic acid, acetic acid, propionic acid, trichloroacetic acid, trifluoroacetic acid, etc.] and an inorganic acid [e.g. hydrochloric acid, hydrobromic acid, sulfuric acid, hydrogen chloride, hydrogen bromide, etc.].

This reaction is usually carried out in a solvent such as methylene chloride, tetrahydrofuran, N,N-dimethylformamide, a mixture thereof or any other solvent which does not adversely influence the reaction.

The reaction temperature is not critical and the reaction is usually carried out at room temperature, under warming or under heating.

The object compound (I) of this invention and pharmaceutically acceptable salts thereof possess strong inhibitory activity against acetylcholinesterase, but hardly possess inhibitory activity against butyrylcholinesterase. That is, the object compound (I) of this invention and pharmaceutically acceptable salts thereof are selective inhibitors of acetylcholinesterase and therefore useful for the treatment of disorders in the central nervous system such as amnesia, dementia [e.g., senile dementia of Alzheimer type, vascular dementia, etc.], cerebrovascular disease or the like.

In order to illustrate the usefulness of the object compound (I), the pharmacological test data of the compound (I) are shown in the following.

[A] Inhibition of acetylcholinesterase

(I) Test Method :

The ability to inhibit acetylcholinesterase was determined by the method (enzyme assay) described in Clinica Chimica Acta, 115 (1981) 163-170. The acetylcholinesterase used in this test was obtained from rat's corpus striatum.

(II) Test Compound :

9-Amino-1-hydroxy-3,3,6-trimethyl-1,2,3,4-tetrahydroacridine

(III) Test Result :

$IC_{50}$ 8.7 x $10^{-7}$ M

[B] Passive avoidance test

(I) Test Method :

6-Weeks old ddY strain male mice (20 per group) were used. The effect of the test compound on deficit of memory induced by cycloheximide (a protein synthesis inhibitor) in passive avoidance task was examined.

The apparatus consists of a grid floor enclosed by a transparent acryl glass wall each section of which was 30 $cm^2$. At the center of this grid a wooden platform of 4 x 4 x 4 cm was fixed. This apparatus was set in a wooden semisoundproof box of 35 x 35 x 90 cm fitted with a 15W tungsten lamp.

Each mouse in groups (A), (B) and (C) was placed on the platform.

When the mouse stepped down from the platform and placed all its paws on the grid floor, an intermittent electrical footshock (1Hz, 0.5 sec, 60V, DC) was delivered for 15 seconds through the grid floor.

Immediately after this training, mice in group (A) [hereinafter referred to as normal mice] were given only vehicle, mice in group (B) [hereinafter referred to as control mice] were given cycloheximide (30 mg/kg, S.C.) and mice in group (C) [hereinafter referred to as drug-treated mice] were given cycloheximide

(30 mg/kg, S.C.) and test compound (i.p.).

24 Hours after the training, each mouse was placed again on the platform and the response latency to step down from the platform was measured to an extent of 300 seconds at the longest.

Results were recorded as the mean response latency of step down for each experimental group of mice.

The effect of the test compound was presented as a percentage of the recovery. The calculation was based on the following equation.

$$\text{Percentage of Recovery (\%)} = \frac{A - B}{C - B} \times 100$$

A : mean response latency of drug-treated mice
B : mean response latency of control mice
C : mean response latency of normal mice

(II) Test Compound

9-Amino-6-chloro-3,3-dimethyl-1,2,3,4-tetrahydroacridine

| (III) Test result : | |
|---|---|
| Percentage of Recovery (%) | |
| Dose (mg/kg) | |
| 0.032 | 95% |

The object compound (I) or its pharmaceutically acceptable salts can usually be administered to mammals including human being in the form of a conventional pharmaceutical composition such as capsule, micro-capsule, tablet, granule, powder, troche, syrup, aerosol, inhalation, solution, injection, suspension, emulsion, or the like.

The pharmaceutical composition of this invention can contain various organic or inorganic carrier materials, which are conventionally used for pharmaceutical purpose, such as excipient (e.g. sucrose, starch, mannit, sorbit, lactose, glucose, cellulose, talc, calcium phosphate, calcium carbonate, etc.), binding agent (cellulose, methyl cellulose, hydroxypropylcellulose, polypropylpyrrolidone, gelatin, gum arabic, polyethyleneglycol, sucrose, starch, etc.), disintegrator (e.g. starch, carboxymethyl cellulose, calcium salt of carboxymethyl cellulose, hydroxypropylstarch, sodium glycole-starch, sodium bicarbonate, calcium phosphate, calcium citrate, etc.), lubricant (e.g. magnesium stearate, talc, sodium laurylsulfate, etc.), flavoring agent (e.g. citric acid, mentol, glycine, orange powders, etc.), preservative (e.g. sodium benzoate, sodium bisulfite, methylparaben, propylparaben, etc.), stabilizer (e.g. citric acid, sodium citrate, acetic acid, etc.), suspending agent (e.g. methyl cellulose, polyvinylpyrrolidone, aluminum stearate, etc.), dispersing agent, aqueous diluting agent (e.g. water), base wax (e.g. cacao butter, polyethyleneglycol, white petrolatum, etc.).

The effective ingredient may usually be administered with a unit dose of 0.01 mg/kg to 10 mg/kg, 1 to 4 times a day. However, the above dosage may be increased or decreased according to age, weight, conditions of the patient or the administering method.

The following preparations and examples are given only for the purpose of illustrating the present invention in more detail.

Preparation 1

To a mixture of 2-aminobenzonitrile (2.0 g) and conc. hydrochloric acid (1.5 ml) in tetrahydrofuran (30 ml) was added dropwise a solution of 5,5-dimethyl-1,3-cyclohexandione (2.37 g) in tetrahydrofuran (15 ml) under reflux for 20 minutes. After the mixture was refluxed for 1 hour and cooled, precipitates were

collected, washed with ethanol and dried in vacuo to give 2-(5,5-dimethyl-3-oxocyclohexen-1-yl)-aminobenzonitrile hydrochloride (3.50 g).
mp : 232-234 °C (dec.)
IR (Nujol) : 1580, 1550 $cm^{-1}$
NMR ($CDCl_3$, $\delta$) : 1.10 (6H, s), 2.36 (2H, s), 2.65 (2H, s), 5.36 (1H, s), 7.42-8.10 (4H, m)
MASS (M/Z) : 240 ($M^+$)

Preparation 2

The following compound was obtained according to a similar manner to that of Preparation 1.

2-(5,5-Dimethyl-3-oxocyclohexen-1-yl)amino-4-trifluoromethylbenzonitrile hydrochloride

mp : 222 °C (dec.)
IR (Nujol) : 1560, 1520 $cm^{-1}$
NMR ($CDCl_3$, $\delta$) : 1.06 (6H, s), 2.22 (2H, s), 2.55 (2H, s), 5.16 (1H, s), 7.80-8.26 (3H, m)
MASS (M/Z) : 308 ($M^+$)

Example 1

A mixture of 2-(5,5-dimethyl-3-oxocyclohexen-1-yl)aminobenzonitrile hydrochloride (3.0 g) copper (II) chloride (0.04 g), potassium carbonate (3.0 g) and tetrahydrofuran (30 ml) was refluxed for 3 hours. After cooling to room temperature, the reaction mixture was evaporated in vacuo. The residue was extracted with ethyl acetate. The extract was washed with water and brine, dried over magnesium sulfate and evaporated in vacuo. The residue was crystallized from ethanol (25 ml) to give 9-amino-3,3-dimethyl-1-oxo-1,2,3,4-tetrahydroacridine (1.6 g).
mp : 221-222 °C
IR (Nujol) : 3300, 3130, 1615, 1550 $cm^{-1}$
NMR ($CDCl_3$, $\delta$) : 1.12 (6H, s), 2.60 (2H, s), 3.03 (2H, s), 7.40-8.04 (4H, m)
MASS (M/Z) : 240 ($M^+$)

| Elemental Analysis $C_{15}H_{16}N_2O$ : | |
|---|---|
| Calcd. : | C 74.97, H 6.71, N 11.66 |
| Found : | C 75.03, H 6.53, N 11.66 |

Example 2

The following compounds were obtained according to a similar manner to that of Example 1.

(1) 9-Amino-3,3-dimethyl-1-oxo-6-trifluoromethyl-1,2,3,4-tetrahydroacridine
mp : 210-212 °C
IR (Nujol) : 3330, 3180, 1645, 1620, 1550 $cm^{-1}$
NMR ($CDCl_3$, $\delta$) : 1.10 (6H, s), 2.63 (2H, s), 2.98 (2H, s), 7.80 (1H, d, J=8Hz), 8.08 (1H, s), 8.66 (1H, d, J=8Hz)

| Elemental Analysis $C_{16}H_{15}F_3N_2O$ : | |
|---|---|
| Calcd. : | C 62.33, H 4.90, N 9.09 |
| Found : | C 61.96, H 4.80, N 8.98 |

Mass (M/Z): 308 ($M^+$)

(2) 9-Amino-3,3,8-trimethyl-1-oxo-1,2,3,4-tetrahydroacridine
mp : 168-170 ° C
IR (Nujol) : 3450, 3150, 1605 $cm^{-1}$
NMR ($DMSO-d_6$, δ) : 1.05 (6H, s), 2.57 (2H, s), 2.87 (2H, s), 2.93 (3H, s), 7.1-7.8 (3H, m)
MASS (M/Z) : 254 ($M^+$)

| Elemental Analysis $C_{16}H_{18}N_2O$ : | |
|---|---|
| Calcd. : | C 75.56, H 7.13, N 11.02 |
| Found : | C 75.30, H 7.04, N 10.97 |

(3) 9-Amino-3,3,7-trimethyl-1-oxo-1,2,3,4-tetrahydroacridine
mp : 242-243 ° C
IR (Nujol) : 3325, 3175, 1610, 825 $cm^{-1}$
NMR ($DMSO-d_6$, δ) : 1.02 (6H, s), 2.43 (3H, s), 2.83 (2H, s), 3.27 (2H, s), 7.4-7.7 (2H, m), 8.0-8.3 (2H, m), 9.7-10.1 (1H, br)
MASS (M/Z) : 254 ($M^+$)

| Elemental Analysis $C_{16}H_{18}N_2O$ : | |
|---|---|
| Calcd. : | C 75.56, H 7.13, N 11.02 |
| Found : | C 75.20, H 7.28, N 11.02 |

(4) 9-Amino-3,3,5-trimethyl-1-oxo-1,2,3,4-tetrahydroacridine
mp : 156-159 ° C
IR (Nujol) : 3375, 3275, 3200, 765 $cm^{-1}$
NMR ($DMSO-d_6$, δ) : 1.07 (6H, s), 2.63 (3H, s), 2.92 (2H, s), 3.33 (1H, s), 7.2-7.8 (3H, m), 8.1-8.6 (2H, m), 9.8-10.1 (1H, br)
MASS (M/Z) : 254 ($M^+$)

| Elemental Analysis $C_{16}H_{18}N_2O$ : | |
|---|---|
| Calcd. : | C 75.56, H 7.13, N 11.02 |
| Found : | C 75.28, H 6.87, N 11.03 |

(5) 9-Amino-3,3,6-trimethyl-1-oxo-1,2,3,4-tetrahydroacridine
mp : 248-250 ° C
IR (Nujol) : 3300, 3150, 1615, 1550, 1480 $cm^{-1}$
NMR ($DMSO-d_6$, δ) : 1.08 (6H, s), 2.50 (3H, s), 2.54 (2H, s), 3.89 (2H, s), 7.26 (1H, d, J=8Hz), 7.48 (1H, s), 8.20 (1H, d, J=8Hz)
MASS (M/Z) : 254 ($M^+$)

Example 3

To a solution of 9-amino-3,3-dimethyl-1-oxo-6-trifluoromethyl-1,2,3,4-tetrahydroacridine (1.0 g) in a mixture of tetrahydrofuran (5 ml) and methanol (5 ml) was added sodium borohydride (0.25 g) under ice cooling and the mixture was stirred for 1 hour at ambient temperature. The reaction mixture was poured into a mixture of water and ethyl acetate. The organic layer was washed with brine, dried over magnesium sulfate and evaporated in vacuo. The residue was crystallized from diethyl ether to give (±)-9-amino-3,3-dimethyl-1-hydroxy-1,2,3,4-tetrahydro-6-trifluoromethylacridine (0.77 g).
mp : 186-188 ° C (dec.)
IR (Nujol) : 3500, 3320, 1600, 1575, 1560, 1500 $cm^{-1}$
NMR ($CDCl_3$, δ) : 0.88 (3H, s), 1.08 (3H, s), 1.82 (1H, d, J=8Hz), 1.99 (1H, d, J=7Hz), 2.72 (2H, s), 4.92

(1H, dd, J=7, 8Hz), 7.00 (2H, s), 7.10 (1H, d, J=8Hz), 8.00 (1H, s), 8.48 (1H, d, J=8Hz)
MASS (M/Z) : 310 ($M^+$)

Example 4

The following compounds were obtained according to a similar manner to that of Example 3.

(1) (±)-9-Amino-3,3-dimethyl-1-hydroxy-1,2,3,4-tetrahydroacridine
mp : 185-186 °C
IR (Nujol) : 3460, 3300, 1600 $cm^{-1}$
NMR (DMSO-$d_6$, δ) : 0.86 (3H, s), 1.06 (3H, s), 1.68 (1H, dd, J=8, 13Hz), 2.00 (1H, dd, J=7, 13Hz), 2.45 (1H, d, J=15Hz), 2.83 (1H, d, J=15Hz), 4.83 (1H, dd, J=7, 8Hz), 5.0-5.2 (1H, br), 6.72 (2H, s), 6.72-8.16 (4H, m)
MASS (M/Z) : 242 ($M^+$)

| Elemental Analysis $C_{15}H_{18}N_2O \cdot 1/8H_2O$ : | |
|---|---|
| Calcd. : | C 73.97, H 7.55, N 11.50 |
| Found : | C 73.62, H 7.46, N 11.45 |

(2) (±)-9-Amino-1-hydroxy-1,2,3,4-tetrahydro-3,3,8-trimethylacridine
mp : 198-199 °C
IR (Nujol) : 3540, 3360, 1610 $cm^{-1}$
NMR (DMSO-$d_6$, δ) : 0.87 (3H, s), 1.10 (3H, s), 1.7-2.3 (2H, m), 2.6-2.8 (2H, m), 2.93 (3H, s), 4.6-5.1 (1H, m), 5.2-5.5 (1H, m), 6.43 (2H, s), 7.0-7.7 (3H, m)
MASS (M/Z) : 256 ($M^+$)

| Elemental Analysis $C_{16}H_{20}N_2O$ : | |
|---|---|
| Calcd. : | C 74.96, H 7.86, N 10.93 |
| Found : | C 74.66, H 7.65, N 10.72 |

(3) (±)-9-Amino-1-hydroxy-1,2,3,4-tetrahydro-3,3,7-trimethylacridine
mp : 200-202 °C
IR (Nujol) : 3475, 3295, 1605, 825 $cm^{-1}$
NMR (DMSO-$d_6$, δ) : 0.86 (3H, s), 1.07 (3H, s), 1.6-2.1 (2H, m), 2.47 (3H, s), 2.67 (2H, s), 4.7-5.5 (2H, br), 6.63 (2H, s), 7.3-8.0 (3H, m)

| Elemental Analysis $C_{16}H_{20}N_2O$ : | |
|---|---|
| Calcd. : | C 74.96, H 7.86, N 10.93 |
| Found : | C 74.54, H 7.60, N 10.78 |

(4) (±)-9-Amino-1-hydroxy-1,2,3,4-tetrahydro-3,3,5-trimethylacridine
mp : 199-201 °C
IR (Nujol) : 3450, 3375, 1630, 760 $cm^{-1}$
NMR (DMSO-$d_6$, δ) : 0.90 (3H, s), 1.10 (3H, s), 1.45-2.2 (2H, m), 2.60 (3H, s), 2.73 (2H, s), 4.6-5.3 (2H, m), 6.67 (2H, s), 7.0-8.1 (3H, m)
MASS (M/Z) : 257 ($M^+$ +1)

| Elemental Analysis $C_{16}H_{20}N_2O$ : | |
|---|---|
| Calcd. : | C 74.96, H 7.86, N 10.93 |
| Found : | C 74.93, H 7.70, N 10.86 |

(5) (±)-9-Amino-1-hydroxy-3,3,6-trimethyl-1,2,3,4-tetrahydroacridine
mp : 205-207 °C (dec.)
IR (Nujol) : 3460, 3320, 1610, 1560, 1495 $cm^{-1}$
NMR (DMSO-$d_6$, δ) : 0.90 (3H, s), 1.10 (3H, s), 1.58-2.14 (2H, m), 2.42 (3H, s), 3.30-3.60 (1H, m), 4.84 (1H, t, J=6Hz), 6.60 (2H, s), 7.14 (1H, d, J=9Hz), 7.40 (1H, s), 8.00 (1H, d, J=9Hz)
MASS (M/Z) : 256 ($M^+$)

Example 5

A mixture of (±)-9-amino-3,3-dimethyl-1-hydroxy-1,2,3,4-tetrahydroacridine (0.5 g) and polyphosphate ester (2.5 g) was heated at 130 °C for 30 minutes under stirring. After cooling to room temperature, the reaction mixture was added to water, stirred for 5 minutes and washed with ethyl acetate (20 ml). The separated aqueous layer was adjusted to pH 10 and extracted with a mixture of ethyl acetate (20 ml) and tetrahydrofuran (20 ml). The organic layer was washed with brine, dried over magnesium sulfate and evaporated in vacuo. Ethanol (5 ml) was added to the residue and then the mixture was added to 20% hydrogen chloride in ethanol (0.5 ml) at 10 °C. The mixture was stirred for 30 minutes at ambient temperature. The precipitate was collected by filtration to give 9-amino-3,4-dihydro-3,3-dimethylacridine hydrochloride (0.28 g).
mp : >300 °C
IR (Nujol) : 3325, 3175, 1660 $cm^{-1}$
NMR ($CF_3COOD$, δ) : 1.27 (6H, s), 3.07 (2H, s), 6.17 (1H, d, J=9Hz), 6.56 (1H, d, J=9Hz), 7.4-8.30 (4H, m)

Example 6

The following compounds were obtained according to a similar manner to that of Example 5.

(1) 9-Amino-3,4-dihydro-3,3,7-trimethylacridine
mp : 208-211 °C
IR (Nujol) : 3375, 3325, 1650, 820 $cm^{-1}$
NMR (DMSO-$d_6$, δ) : 1.03 (6H, s), 2.47 (3H, s), 2.73 (2H, s), 5.77 (1H, d, J=10Hz), 6.52 (2H, s), 6.83 (1H, d, J=10Hz), 7.3-8.0 (3H, s)
MASS (M/Z) : 238 ($M^+$)

(2) 9-Amino-3,4-dihydro-3,3,5-trimethylacridine
mp : 156-158 °C
IR (Nujol) : 3480, 3375, 3200, 1640 $cm^{-1}$
NMR (DMSO-$d_6$, δ) : 1.07 (6H, s), 2.60 (3H, s), 2.77 (2H, s), 5.70 (1H, d, J=9Hz), 6.80 (1H, d, J=9Hz), 7.0-7.4 (2H, m), 7.8-8.0 (1H, m)
MASS (M/Z) : 238 ($M^+$)

(3) 9-Amino-3,4-dihydro-3,3-dimethyl-6-trifluoromethylacridine hydrochloride
mp: 286 °C (dec.)
IR (Nujol) : 3300, 3140, 1675, 1605, 1585, 1555, 1505 $cm^{-1}$
NMR (DMSO-$d_6$, δ) : 1.10 (6H, s), 3.06 (2H, s), 5.96 (1H, d, J=10Hz), 6.88 (1H, d, J=10Hz), 7.88 (1H, d, J=9Hz), 8.45 (1H, s), 8.75 (1H, d, J=9Hz), 8.81 (2H, s)
MASS (M/Z) : 292 ($M^+$)

(4) 9-Amino-3,3,6-trimethyl-3,4-dihydroacridine
mp : 190-192 °C
IR (Nujol) : 3450, 3310, 1650, 1615 $cm^{-1}$
NMR (DMSO-$d_6$, δ) : 1.06 (6H, s), 2.42 (3H, s), 2.76 (2H, s), 5.60 (1H, d, J=6Hz), 6.55 (2H, br), 6.82 (1H, d, J=6Hz), 7.16 (1H, d, J=6Hz), 7.46 (1H, s), 8.04 (1H, d, J=6Hz)
MASS (M/Z) : 238 ($M^+$)

Example 7

A mixture of 9-amino-3,4-dihydro-3,3-dimethylacridine hydrochloride (0.8 g) and 10% palladium-carbon (0.4 g) in acetic acid (20 ml) was hydrogenated at room temperature under atmospheric pressure for 2 hours. After removal of the catalyst by filtration, the filtrate was evaporated under reduced pressure. The residue was extracted with ethyl acetate (50 ml), washed with 10% aqueous potassium carbonate and brine, dried over magnesium sulfate and evaporated in vacuo. Ethanol (8 ml) was added to the residue and then the mixture was added to 20% hydrogen chloride in ethanol (1 ml) at 10 °C. The mixture was stirred for 30 minutes at ambient temperature. The precipitate was collected by filtration to give 9-amino-3,3-dimethyl-1,2,3,4-tetrahydroacridine hydrochloride (0.45 g).
mp : 191-193 °C
IR (Nujol) : 3490, 3310, 1645 $cm^{-1}$
NMR (DMSO-$d_6$, δ) : 1.00 (6H, s), 1.32 (2H, t, J=7Hz), 2.60 (2H, t, J=7Hz), 2.63 (2H, s), 7.2-7.8 (3H, m), 8.1-8.3 (1H, m)

Example 8

The following compounds were obtained according to a similar manner to that of Example 7.
(1) 9-Amino-1,2,3,4-tetrahydro-3,3,7-trimethylacridine
mp : 238-239 °C
IR (Nujol) : 3375, 3130, 1650, 1565 $cm^{-1}$
NMR (DMSO-$d_6$, δ) : 0.99 (6H, s), 1.67 (2H, t, J=8Hz), 2.48 (3H, s), 3.52 (2H, t, J=8Hz), 6.27 (2H, s), 7.3-8.0 (3H, m)
MASS (M/Z) : 240 ($M^+$)
(2) 9-Amino-1,2,3,4-tetrahydro-3,3,5-trimethylacridine
mp : 157-158 °C
IR (Nujol) : 3475, 3300, 1635 $cm^{-1}$
NMR (DMSO-$d_6$, δ) : 0.97 (6H, s), 1.62 (2H, t, J=7Hz), 2.57 (2H, t, J=7Hz), 2.60 (3H, s), 2.67 (2H, s), 6.15 (2H, s), 7.03-7.37 (2H, m), 7.93 (1H, d, J=9Hz)
(3) 9-Amino-3,3-dimethyl-1,2,3,4-tetrahydro-6-trifluoromethylacridine
mp : 245-246 °C
IR (Nujol) : 3500, 3320, 3180, 1640, 1580, 1560, 1500 $cm^{-1}$
NMR (DMSO-$d_6$, δ) : 0.95 (6H, s), 1.62 (2H, t, J=7Hz), 2.50-2.65 (4H, m), 6.54 (2H, s), 7.46 (1H, d, J=9Hz), 7.88 (1H, s), 8.32 (1H, d, J=9Hz)
(4) 9-Amino-3,3,6-trimethyl-1,2,3,4-tetrahydroacridine
mp : 203-204 °C
IR (Nujol) : 3500, 3310, 3150, 1640, 1580, 1560, 1500 $cm^{-1}$
NMR (DMSO-$d_6$, δ) : 0.98 (6H, s), 1.66 (2H, t, J=7Hz), 2.46 (3H, s), 2.60-2.73 (4H, m), 6.32 (2H, br), 7.20 (1H, dd, J=2, 8Hz), 7.46 (1H, d, J=2Hz), 8.08 (1H, d, J=8Hz)
MASS (M/Z) : 240 ($M^+$)

| Elemental Analysis $C_{16}H_{20}N_2 \cdot 1/4H_2O$ : | |
|---|---|
| Calcd. : | C 78.49, H 8.43, N 11.44 |
| Found : | C 78.41, H 8.38, N 11.33 |

Preparation 3

The following compounds were obtained according to a similar manner to that of Preparation 1.
(1) 2-(5,5-Dimethyl-3-oxocyclohexen-1-yl)amino-6-fluorobenzonitrile hydrochloride
mp : 208-210 °C
IR (Nujol) : 2230, 1700, 1615, 1540 $cm^{-1}$
NMR (DMSO-$d_6$, δ) : 1.07 (6H, s), 2.23 (2H, s), 2.54 (2H, s), 5.26 (1H, s), 7.36 (1H, d, J=8Hz), 7.46 (1H, dd,

J = 8Hz), 7.85 (1H, dd, J = 8Hz, 15Hz)
MASS (M/Z) : 258 ($M^+$)

(2) 2-(5,5-Dimethyl-3-oxocyclohexen-1-yl)amino-4-chlorobenzonitrile
mp : 184-186 °C
IR (Nujol) : 3250, 3160, 2220, 1600, 1560 $cm^{-1}$
NMR (DMSO-$d_6$, δ) : 1.05 (6H, s), 2.08 (2H, s), 2.51 (2H, s), 4.92 (1H, s), 7.49-7.56 (2H, m), 7.93 (1H, d, J = 8Hz), 9.12 (1H, s)
MASS (M/Z) : 274 ($M^+$)

(3) 2-(5,5-Dimethyl-3-oxocyclohexen-1-yl)amino-4-methoxybenzonitrile hydrochloride
mp : 220-221 °C
IR (Nujol) : 2220, 1610, 1560, 1520 $cm^{-1}$
NMR (DMSO-$d_6$, δ) : 1.07 (6H, s), 2.28 (2H, s), 2.56 (2H, s), 3.87 (3H, s), 5.31 (1H, s), 7.04 (1H, d, J = 2Hz), 7.11 (1H, dd, J = 2Hz, 8Hz), 7.89 (1H, d, J = 8Hz)
MASS (M/Z) : 270 ($M^+$)

## Preparation 4

A suspension of 2-amino-4-fluorobenzonitrile (5.00 g) and p-toluenesulfonic acid monohydrate (0.21 g) in toluene (30 ml) was refluxed, and the water was collected in a Dean-Stark water separator. After 2 hours, the mixture was poured into water (50 ml) and extracted with ethyl acetate (50 ml). The separated organic layer was washed with water and brine, dried over magnesium sulfate and evaporated in vacuo. The residue was recrystallized from ether to give 2-(5,5-dimethyl-3-oxocyclohexen-1-yl)amino-4-fluorobenzonitrile (7.44 g).
mp : 263-264 °C
IR (Nujol) : 3180, 2240, 1600 $cm^{-1}$
NMR (DMSO-$d_6$, δ) : 1.05 (6H, s), 2.09 (2H, s), 2.41 (2H, s), 4.97 (1H, s), 7.26-7.39 (2H, m), 7.99 (1H, dd, J = 6.1Hz, 8.6Hz), 9.14 (1H, s)
MASS (M/Z) : 258 ($M^+$)

## Example 9

A mixture of 2-amino-4-chlorobenzonitrile (10.48 g), 3,3-dimethylhexanone (13.00 g) and zinc chloride (18.72 g) in N,N-dimethylformamide (25 ml) was stirred at 130 °C. After 10 hours, the reaction mixture was poured into water (100 ml). The precipitate was collected by filtration and washed with ethyl acetate. The suspension of the precipitate in a mixture of water (200 ml), ethyl acetate (1 ℓ) and tetrahydrofuran (1 ℓ) was adjusted to pH 10.0 with 4N aqueous sodium hydroxide. The separated organic layer was washed with brine, dried over magnesium sulfate and evaporated in vacuo. The residue was recrystallized from ether to give 9-Amino-6-chloro-3,3-dimethyl-1,2,3,4-tetrahydroacridine (11.44 g).
mp : 211-212 °C
IR (Nujol) : 3480, 3300, 3120, 1640, 1605, 1560 $cm^{-1}$
NMR (DMSO-$d_6$, δ) : 0.97 (6H, s), 1.66 (2H, t, J = 6Hz), 2.55 (2H, t, J = 6Hz), 2.62 (2H, s), 6.51 (2H, s), 7.30 (1H, dd, J = 2Hz, 9Hz), 7.64 (1H, d, J = 2Hz), 8.20 (1H, d, J = 9Hz)
MASS (M/Z) : 260 ($M^+$)

| Elemental Analysis $C_{15}H_{17}ClN_2$ : | |
|---|---|
| Calcd. : | C 69.09, H 6.57, N 10.74, Cl 13.59 |
| Found : | C 68.86, H 6.42, N 10.66, Cl 13.90 |

## Example 10

The following compounds were obtained according to a similar manner to that of Example 9.

(1) 9-Amino-3,3-dimethyl-6-fluoro-1,2,3,4-tetrahydroacridine

mp : 209-211 °C
IR (Nujol) : 3500, 3320, 3120, 1640, 1580, 1500 $cm^{-1}$
NMR ($CDCl_3$, δ) : 1.04 (6H, s), 1.73 (2H, t, J=7Hz), 2.61 (2H, t, J=7Hz), 2.78 (2H, s), 4.73 (2H, br), 7.12 (1H, ddd, J=2Hz, 8Hz, 10Hz), 7.50 (1H, dd, J=2Hz, 10Hz), 7.68 (1H, dd, J=6Hz, 8Hz)
MASS (M/Z) : 244 ($M^+$)

| Elemental Analysis $C_{15}H_{17}FN_2$ : | |
|---|---|
| Calcd. : | C 73.74, H 7.01, N 11.46 |
| Found : | C 73.30, H 6.97, N 11.37 |

(2) 9-Amino-3,3-dimethyl-6-methoxy-1,2,3,4-tetrahydroacridine
mp : 222-223 °C
IR (Nujol) : 3480, 3300, 3110, 1640, 1620, 1570, 1560 $cm^{-1}$
NMR (DMSO-$d_6$, δ) : 0.97 (6H, s), 1.62 (2H, t, J=7Hz), 2.54 (2H, t, J=7Hz), 2.59 (2H, s), 3.83 (3H, s), 6.27 (2H, s), 6.92 (1H, dd, J=2Hz, 9Hz), 7.03 (1H, d, J=2Hz), 8.05 (1H, d, J=9Hz)
MASS (M/Z) : 256 ($M^+$)

| Elemental Analysis $C_{16}H_{20}N_2O \cdot 0.2H_2O$ : | |
|---|---|
| Calcd. : | C 73.92, H 7.90, N 10.77 |
| Found : | C 74.00, H 7.77, N 10.43 |

(3) 9-Amino-6-chloro-3,3-dimethyl-1,2,3,4-tetrahydroacridine hydrochloride
mp : 280 °C (dec.)
IR (Nujol) : 3350, 3200, 3140, 1660, 1590 $cm^{-1}$
NMR (DMSO-$d_6$, δ) : 1.00 (6H, s), 1.65 (2H, t, J=6Hz), 2.55 (2H, t, J=6Hz), 2.82 (2H, s), 7.66 (1H, d, J=9Hz), 8.10 (1H, s), 8.61 (1H, d, J=9Hz), 8.30-9.10 (2H, br)

(4) 9-Amino-8-chloro-3,3-dimethyl-1,2,3,4-tetrahydroacridine
mp : 164-165 °C
IR (Nujol) : 3480, 3300, 3280, 1630, 1600, 1540 $cm^{-1}$
NMR ($CDCl_3$, δ) : 1.04 (6H, s), 1.74 (2H, t, J=7Hz), 2.52 (2H, t, J=7Hz), 2.76 (2H, s), 5.83 (2H, br), 7.27-7.41 (2H, m), 7.69 (1H, dd, J=2Hz, 8Hz)
MASS (M/Z) : 260 ($M^+$)

(5) 9-Amino-6-chloro-2,2-dimethyl-1,2,3,4-tetrahydroacridine
mp : 220-222 °C
IR (Nujol) : 3460, 3300, 1650, 1605, 1560 $cm^{-1}$
NMR (DMSO-$d_6$, δ) : 1.03 (6H, s), 1.62 (2H, t, J=7Hz), 2.35 (2H, s), 2.85 (2H, t, J=7Hz), 6.49 (2H, s), 7.28 (1H, dd, J=2Hz, 9Hz), 7.63 (1H, d, J=2Hz), 8.19 (1H, d, J=9Hz)
MASS (M/Z) : 260 ($M^+$)

(6) 9-Amino-2,2-dimethyl-8-fluoro-1,2,3,4-tetrahydroacridine
mp : 215-217 °C
IR (Nujol) : 3550, 3300, 3200, 1625 $cm^{-1}$
NMR (DMSO-$d_6$, δ) : 1.03 (6H, s), 1.63 (2H, t, J=6.8Hz), 2.33 (2H, s), 2.86 (2H, t, J=6.8Hz), 6.17 (2H, s), 7.0-7.1 (1H, m), 7.4-7.6 (2H, m)

(7) (-)-9-Amino-6-chloro-3,3-dimethyl-1,2,3,4-tetrahydro-2,4-methanoacridine hydrochloride
mp : 215 °C
IR (Nujol) : 3350, 3210, 1630, 1600, 1585 $cm^{-1}$
NMR (DMSO-$d_6$, δ) : 0.72 (3H, s), 1.39-1.45 (4H, m), 2.46-2.49 (1H, m), 2.62-2.88 (3H, m), 2.23 (1H, t, J=5Hz), 7.71 (1H, dd, J=2Hz, 9Hz), 8.14 (1H, d, J=2Hz), 8.59 (1H, d, J=9Hz)
MASS (M/Z) : 272 ($M^+$)
$[\alpha]_D^{20}$ : -63.2° (C=0.5, $CH_3OH$)

(8) 9-Amino-2,2-dimethyl-6-methoxy-1,2,3,4-tetrahydroacridine
mp : 234-235 °C

IR (Nujol) : 3380, 3320, 3160, 1640, 1615, 1570, 1500 $cm^{-1}$
NMR ($DMSO-d_6$, δ) : 1.02 (6H, s), 1.61 (2H, t, J=6.8Hz), 2.33 (2H, s), 2.82 (2H, t, J=6.8Hz), 3.82 (3H, s), 6.28 (2H, s), 6.90 (1H, dd, J=2.6Hz, 9.1Hz), 7.02 (1H, d, J=2.6Hz), 8.04 (1H, d, J=9.1Hz)
MASS (M/Z) : 256 ($M^+$)

Example 11

The following compounds were obtained according to a similar manner to that of Example 1.

(1) 9-Amino-3,3-dimethyl-6-methoxy-1-oxo-1,2,3,4-tetrahydroacridine
mp : 290-291 °C
IR (Nujol) : 3300, 3150, 1740, 1620, 1560 $cm^{-1}$
NMR ($DMSO-d_6$, δ) : 1.02 (6H, s), 2.49 (2H, s), 2.83 (2H, s), 3.88 (3H, s), 7.05 (1H, dd, J=2Hz, 9Hz), 7.11 (1H, d, J=2Hz), 8.23 (1H, d, J=9Hz), 8.21-8.25 (1H, br), 9.86 (1H, br)
MASS (M/Z) : 270 ($M^+$)

(2) 9-Amino-6-chloro-3,3-dimethyl-1-oxo-1,2,3,4-tetrahydroacridine
mp : 255-256 °C
IR (Nujol) : 3280, 3100, 1610, 1560 $cm^{-1}$
NMR ($DMSO-d_6$, δ) : 1.03 (6H, s), 2.53 (2H, s), 2.87 (2H, s), 7.48 (1H, dd, J=2Hz, 9Hz), 7.72 (1H, d, J=2Hz), 8.38 (1H, d, J=9Hz), 8.51 (1H, br), 9.99 (1H, br)
MASS (M/Z) : 274 ($M^+$)

(3) 9-Amino-3,3-dimethyl-8-fluoro-1-oxo-1,2,3,4-tetrahydroacridine
mp : 138-139 °C
IR (Nujol) : 3420, 3320, 3180, 1740, 1600, 1560, 1540 $cm^{-1}$
NMR ($CDCl_3$, δ) : 1.15 (6H, s), 2.57 (2H, s), 2.95 (2H, s), 6.96-7.08 (2H, m), 7.52-7.65 (2H, m), 10.54 (1H, br)
MASS (M/Z) : 258 ($M^+$)

(4) 9-Amino-3,3-dimethyl-6-fluoro-1-oxo-1,2,3,4-tetrahydroacridine
mp : 247-248 °C
IR (Nujol) : 3300, 3150, 1620, 1580 $cm^{-1}$
NMR ($DMSO-d_6$, δ) : 1.03 (6H, s), 2.52 (2H, s), 2.86 (2H, s), 7.31-7.45 (2H, m), 8.40-8.48 (2H, m), 10.00 (1H, s)
MASS (M/Z) : 258 ($M^+$)

Example 12

The following compounds were obtained according to a similar manner to that of Example 3.

(1) (±)-9-Amino-3,3-dimethyl-8-fluoro-1-hydroxy-1,2,3,4-tetrahydroacridine
mp : 203-204 °C
IR (Nujol) : 3500, 3300, 3080, 1600, 1570 $cm^{-1}$
NMR ($DMSO-d_6$, δ) : 0.87 (3H, s), 1.08 (3H, s), 1.70 (1H, dd, J=8Hz, 13Hz), 2.01 (1H, dd, J=7Hz, 13Hz), 2.53 (1H, d, J=15Hz), 2.72 (1H, d, J=15Hz), 4.81 (1H, ddd, J=7Hz, 7Hz, 8Hz), 6.68 (2H, s), 7.01-7.12 (1H, m), 7.41-7.50 (2H, m)
MASS (M/Z) : 260 ($M^+$)

(2) (±)-9-Amino-3,3-dimethyl-6-fluoro-1-hydroxy-1,2,3,4-tetrahydroacridine
mp : 211-212 °C (dec.)
IR (Nujol) : 3360, 3260, 1640, 1620, 1575 $cm^{-1}$
NMR ($DMSO-d_6$, δ) : 0.87 (3H, s), 1.07 (3H, s), 1.70 (1H, dd, J=8Hz, 13Hz), 2.00 (1H, dd, J=8Hz, 13Hz), 2.53 (1H, d, J=16Hz), 2.71 (1H, d, J=16Hz), 4.85 (1H, t, J=8Hz), 5.26 (1H, s), 6.85 (2H, s), 7.23 (1H, ddd, J=3Hz, 9Hz, 11Hz), 7.34 (1H, dd, J=3Hz, 11Hz), 8.26 (1H, dd, J=6Hz, 9Hz)
MASS (M/Z) : 260 ($M^+$)

Example 13

The following compounds were obtained according to a similar manner to that of Example 5.

(1) 9-Amino-3,4-dihydro-3,3-dimethyl-6-methoxyacridine hydrochloride

mp : 235 °C (dec.)
IR (Nujol) : 3450, 3420, 3300, 3160, 1680, 1630 $cm^{-1}$
NMR (DMSO-$d_6$, δ) : 1.10 (6H, s), 2.97 (2H, s), 3.92 (3H, s), 5.89 (1H, d, J=9.8Hz), 6.86 (1H, d, J=9.8Hz), 7.26 (1H, dd, J=9.3Hz, 2.5Hz), 7.41 (1H, dd, J=2.5Hz), 8.45 (1H, d, J=9.3Hz), 8.42-8.47 (1H, br), 14.26 (1H, br)
MASS (M/Z) : 254 ($M^+$)

| Elemental Analysis $C_{16}H_{18}N_2O \cdot HCl \cdot H_2O$ : | |
|---|---|
| Calcd. : | C 62.23, H 6.85, N 9.07 |
| Found : | C 62.67, H 6.86, N 8.91 |

(2) 9-Amino-6-chloro-3,4-dihydro-3,3-dimethylacridine hydrochloride
mp : 308-309 °C
IR (Nujol) : 3320, 3150, 2750, 1655, 1630, 1600, 1570 $cm^{-1}$
NMR (DMSO-$d_6$, δ) : 1.10 (6H, s), 3.04 (2H, s), 5.96 (1H, d, J=10Hz), 6.89 (1H, d, J=10Hz), 7.70 (1H, dd, J=2Hz, 7Hz), 8.15 (1H, d, J=2Hz), 8.60 (1H, d, J=7Hz), 8.80-9.00 (2H, br)
MASS (M/Z) : 258 ($M^+$)

| Elemental Analysis $C_{15}H_{15}ClN_2 \cdot HCl \cdot 0.5H_2O$ : | |
|---|---|
| Calcd. : | C 59.22, H 5.63, N 9.20 |
| Found : | C 59.38, H 5.34, N 9.01 |

(3) 9-Amino-3,4-dihydro-3,3-dimethyl-8-fluoroacridine
mp : 187-188 °C
IR (Nujol) : 3500, 3300, 3150, 1640, 1620, 1560 $cm^{-1}$
NMR (DMSO-$d_6$, δ) : 1.05 (6H, s), 2.74 (2H, s), 5.80 (1H, d, J=10Hz), 6.48-6.49 (2H, br), 6.85 (1H, d, J=10Hz), 7.03-7.15 (1H, m), 7.41-7.53 (2H, m)
MASS (M/Z) : 242 ($M^+$)

(4) 9-Amino-3,4-dihydro-3,3,6-trimethylacridine hydrochloride
IR (Nujol) : 3320, 3130, 1660, 1580 $cm^{-1}$
NMR (DMSO-$d_6$, δ) : 1.10 (6H, s), 2.51 (3H, s), 3.02 (2H, s), 5.91 (1H, d, J=9.8Hz), 6.89 (1H, d, J=9.8Hz), 7.47 (1H, d, J=7.4Hz), 7.82 (1H, s), 8.43 (1H, d, J=7.4Hz), 8.15-8.9 (2H, br), 14.5 (1H, s)

| Elemental Analysis $C_{16}H_{18}N_2 \cdot HCl \cdot 1/4H_2O$ : | |
|---|---|
| Calcd. : | C 68.80, H 7.03, N 10.02 |
| Found : | C 68.71, H 7.09, N 9.92 |

(5) 9-Amino-3,4-dihydro-3,3-dimethyl-6-fluoroacridine
mp : 178-180 °C
IR (Nujol) : 3490, 3310, 3100, 1645, 1620 $cm^{-1}$
NMR (DMSO-$d_6$, δ) : 1.05 (6H, s), 2.75 (2H, s), 5.75 (1H, d, J=10Hz), 6.83 (1H, d, J=10Hz), 7.25 (1H, ddd, J=3Hz, 9Hz, 11Hz), 7.39 (1H, dd, J=3Hz, 11Hz), 8.25 (1H, dd, J=6Hz, 9Hz)
MASS (M/Z) : 242 ($M^+$)

Example 14

(±)-9-Amino-1-hydroxy-1,2,3,4-tetrahydro-3,3,6-trimethylacridine (4 g) and (+)-Di-p-toluoyl-D-tartaric

acid (6.3 g) were dissolved with ethanol (66 ml) at 60° C, then the solution was allowed to stand overnight at ambient temperature. The precipitate was collected by filtration and suspended with dichloromethane (100 ml) and water (100 ml). The solution was adjusted to pH 13 with 4N aqueous sodium hydroxide solution, and the organic layer was washed with water and brine, dried over magnesium sulfate and evaporated in vacuo. The residue was recrystallized from diethyl ether to give (+)-9-amino-1-hydroxy-1,2,3,4-tetrahydro-3,3,6-trimethylacridine.

mp : 207-208° C (dec.)

$[\alpha]_D^{20}$ = +136.0° (C=0.5, $CH_3OH$)

IR (Nujol) : 3475, 3330, 1610, 770 $cm^{-1}$

NMR (DMSO-$d_6$, δ) : 0.86 (3H, s), 1.07 (3H, s), 1.68 (1H, dd, J=13Hz, 8.3Hz), 1.98 (dd, J=6.6Hz, 13Hz), 2.42 (3H, s), 2.65 (2H, m), 4.81 (1H, m), 5.17 (1H, d, J=6.4Hz), 6.66 (2H, s), 7.13 (1H, d, J=8.5Hz), 7.42 (1H, s), 8.02 (1H, d, J=8.5Hz)

| Elemental Analysis $C_{16}H_{20}N_2O \cdot 1/2H_2O$ : | |
|---|---|
| Calcd. : | C 72.42, H 7.97, N 10.55 |
| Found : | C 72.92, H 7.76, N 10.56 |

Example 15

The following compound was obtained by treating (±)-9-amino-1-hydroxy-1,2,3,4-tetrahydro-3,3,6-trimethylacridine with (-)-Di-p-toluoyl-D-tartaric acid according to a similar manner to that of Example 14.

(-)-9-Amino-1-hydroxy-1,2,3,4-tetrahydro-3,3,6-trimethylacridine

mp : 210-211° C (dec.)

$[\alpha]_D^{20}$ = -105.2° (C=0.5, $CH_3OH$)

IR (Nujol) : 3460, 3400, 3300, 1610 $cm^{-1}$

NMR (DMSO-$d_6$, δ) : 0.86 (3H, s), 1.07 (3H, s), 1.68 (1H, dd, J=13Hz, 8.3Hz), 1.98 (dd, J=13Hz, 6.6Hz), 2.42 (3H, s), 2.65 (2H, m), 4.83 (1H, m), 5.21 (1H, m), 6.66 (2H, s), 7.13 (1H, d, J=8.5Hz), 7.42 (1H, s), 8.02 (1H, d, J=8.5Hz)

| Elemental Analysis $C_{16}H_{20}N_2O$ : | |
|---|---|
| Calcd. : | C 74.96, H 7.86, N 10.92 |
| Found : | C 74.78, H 7.87, N 10.74 |

Example 16

To a solution of citric acid monohydrate (0.81 g) in ethanol (10 ml) was added 9-amino-6-chloro-3,3-dimethyl-1,2,3,4-tetrahydroacridine (1.00 g). The solution was allowed to stand for 1 hour at ambient temperature. The crystal was collected by filtration and dried in vacuo to give 9-amino-6-chloro-3,3-dimethyl-1,2,3,4-tetrahydroacridine citrate (1.42 g).

mp : 210-211° C

IR (Nujol) : 3360, 3250, 3120, 1720, 1670, 1640, 1600, 1650 $cm^{-1}$

NMR (DMSO-$d_6$, δ) : 1.02 (6H, s), 1.67 (2H, t, J=7Hz), 2.49-2.59 (7H, m), 2.72 (2H, s), 7.60 (1H, dd, J=2Hz, 9Hz), 7.80 (1H, d, J=2Hz), 8.19 (2H, s), 8.43 (1H, d, J=9Hz)

MASS (M/Z) : 260 ($M^+$)

| Elemental Analysis $C_{15}H_{17}ClN_2 \cdot C_6H_8O_7 \cdot 0.2H_2O$ : | |
|---|---|
| Calcd. : | C 55.25, H 5.60, N 6.13, Cl 7.76 |
| Found : | C 55.13, H 5.48, N 5.97, Cl 7.78 |

Example 17

The following compounds were obtained according to a similar manner to that of Example 16.

(1) 9-Amino-2,2-dimethyl-8-fluoro-1,2,3,4-tetrahydroacridine L-(+)-tartrate

mp : 190-191 °C

IR (Nujol) : 3350, 3240, 1640, 1600, 810 $cm^{-1}$

NMR (DMSO-$d_6$, δ) : 1.04 (6H, s), 1.63 (2H, t, J=6.6Hz), 2.33 (2H, s), 2.96 (2H, t, J=6.6Hz), 4.14 (2H, s), 7.18-7.4 (1H, m), 7.39 (2H, m), 7.6-7.7 (2H, m), 8.07 (4H, br)

(2) 9-Amino-3,4-dihydro-3,3-dimethyl-8-fluoroacridine citrate

mp : 168-169 °C

IR (Nujol) : 3480, 3420, 3340, 3250, 1740, 1720, 1640, 1580 $cm^{-1}$

NMR (DMSO-$d_6$, δ) : 1.07 (6H, s), 2.51-2.74 (4H, m), 2.80 (2H, s), 5.87 (1H, d, J=9.8Hz), 6.87 (1H, d, J=9.8Hz), 7.17-7.28 (3H, m), 7.54-7.67 (2H, m)

MASS (M/Z) : 242 ($M^+$)

| Elemental Analysis $C_{15}H_{15}FN_2 \cdot C_6H_8O_7$ : | |
|---|---|
| Calcd. : | C 58.06, H 5.33, N 6.44 |
| Found : | C 57.86, H 5.42, N 6.41 |

(3) 9-Amino-8-chloro-3,3-dimethyl-1,2,3,4-tetrahydroacridine citrate

mp : 208-209 °C (dec.)

IR (Nujol) : 3420, 3290, 3240, 3190, 1710, 1630, 1590 $cm^{-1}$

NMR (DMSO-$d_6$, δ) : 1.01 (6H, s), 1.68 (2H, t, J=6.6Hz), 2.50-2.69 (6H, m), 2.70 (2H, s), 7.54-7.77 (5H, m)

MASS (M/Z) : 260 ($M^+$)

| Elemental Analysis $C_{15}H_{17}ClN_2 \cdot C_6H_8O_7$ : | |
|---|---|
| Calcd. : | C 55.69, H 5.56, N 6.18 |
| Found : | C 55.60, H 5.72, N 6.12 |

Example 18

A mixture of 2-amino-6-fluorobenzonitrile (2.00 g), 3,3-dimethylhexanone (2.78 g) and zinc chloride (4.00 g) in N,N-dimethylformamide (5 ml) was stirred at 130 °C. After 7.5 hours, the reaction mixture was poured into water. The precipitate was collected by filtration and washed with ethyl acetate. The suspension of the precipitate in a mixture of water and ethyl acetate was adjusted to pH 9.5 with 4N aqueous sodium hydroxide. The separated organic layer was washed with brine, dried over magnesium sulfate and evaporated in vacuo. The residue was recrystallized from ether to give 9-amino-3,3-dimethyl-8-fluoro-1,2,3,4-tetrahydroacridine (2.58 g). This compound (1.0 g) and citric acid monohydrate (0.86 g) were dissolved in ethanol (10 ml). After the mixture was allowed to stand at ambient temperature, the precipitate was collected and dried to give 9-amino-3,3-dimethyl-8-fluoro-1,2,3,4-tetrahydroacridine citrate (1.51 g).

mp : 100-101 °C
IR (Nujol) : 3350, 3250, 1720, 1650, 1600, 1500 $cm^{-1}$
NMR (DMSO-$d_6$, δ) : 1.01 (6H, s), 1.67 (2H, t, J=7Hz), 2.50-2.60 (6H, m), 2.72 (2H, s), 7.32 (1H, dd, J=8Hz, 13Hz), 7.61 (1H, d, J=8Hz), 7.67-7.80 (3H, m)
MASS (M/Z) : 244 ($M^+$)

| Elemental Analysis $C_{21}H_{25}FN_2O_7 \cdot 0.5H_2O$ : | |
|---|---|
| Calcd. : | C 56.62, H 5.88, N 6.28 |
| Found : | C 56.56, H 5.87, N 6.27 |

Example 19

The following compound was obtained according to a similar manner to that of Example 18.

9-Amino-3,3,8-trimethyl-1,2,3,4-tetrahydroacridine citrate

mp : 198-200 °C
IR (Nujol) : 3475, 1710 $cm^{-1}$
NMR (DMSO-$d_6$, δ) : 1.02 (6H, s), 1.68 (2H, t, J=6.4Hz), 2.5-2.7 (6H, m), 2.74 (2H, s), 2.94 (3H, s), 7.3-7.4 (1H, m), 7.6-7.8 (4H, m)

Example 20

To a mixture of 9-amino-1,2,3,4-tetrahydro-1-oxo-3,3-dimethyl-6-methoxyacridine (1.5 g) and sodium borohydride (0.42 g) in tetrahydrofuran (25 ml) was added methanol (5 ml) dropwise under reflux. After the mixture was refluxed for 2 hours, the reaction mixture was poured into water and extracted with ethyl acetate. The separated organic layer was washed with brine, dried over magnesium sulfate and evaporated in vacuo. The residue (0.8 g) and maleic acid (0.34 g) were dissolved in ethanol (10 ml). The mixture was evaporated in vacuo and the residue was recrystallized from ether to give (±)-9-amino-3,3-dimethyl-1-hydroxy-6-methoxy-1,2,3,4-tetrahydroacridine maleate (0.9 g).
mp : 186-187 °C
IR (Nujol) : 3400, 3300, 3200, 1640 $cm^{-1}$
NMR (DMSO-$d_6$, δ) : 0.98 (3H, s), 1.13 (3H, s), 1.75 (1H, dd, J=7Hz, 13Hz), 2.02 (1H, dd, J=7Hz, 13Hz), 2.56 (1H, d, J=13Hz), 2.84 (1H, d, J=13Hz), 3.94 (3H, s), 4.88 (1H, br), 5.53 (1H, br), 6.07 (2H, s), 7.16 (1H, d, J=2Hz), 7.26 (1H, dd, J=2Hz, 9Hz), 8.41 (1H, d, J=9Hz)
MASS (M/Z) : 272 ($M^+$)

| Elemental Analysis $C_{20}H_{24}N_2O_6$ : | |
|---|---|
| Calcd. : | C 61.84, H 6.22, N 7.21 |
| Found : | C 61.70, H 6.32, N 6.90 |

Example 21

The following compound was obtained according to a similar manner to that of Example 20.

(±)-9-Amino-6-chloro-3,3-dimethyl-1-hydroxy-1,2,3,4-tetrahydroacridine maleate

mp : 140-144° C
IR (Nujol) : 3380, 3200, 3100, 1640, 1560 $cm^{-1}$
NMR (DMSO-$d_6$, δ) : 0.95 (3H, s), 1.12 (3H, s), 1.74 (1H, dd, J=7Hz, 13Hz), 2.02 (1H, dd, J=7Hz, 13Hz), 2.63 (1H, d, J=17Hz), 2.83 (1H, dd, J=17Hz), 4.88 (1H, t, J=7Hz), 6.06 (2H, s), 7.61 (1H, dd, J=9Hz, 2Hz), 7.77 (1H, d, J=2Hz), 8.25 (2H, s), 8.45 (1H, d, J=9Hz)
MASS (M/Z) : 276 ($M^+$)

| Elemental Analysis $C_{19}H_{21}ClN_2O_5 \cdot 0.25H_2O$ : | |
|---|---|
| Calcd. : | C 57.43, H 5.45, N 7.05, Cl 8.92 |
| Found : | C 57.31, H 5.68, N 7.42, Cl 8.03 |

Example 22

A suspension of 9-amino-3,3-dimethyl-6-methoxy-1,2,3,4-tetrahydroacridine (1.0 g) in 48% aqueous hydrogen bromide (30 ml) was refluxed for 5 hours. After cooling to room temperature, the precipitate was collected and suspended in water (20 ml). The suspension was adjusted to pH 8.0 with saturated aqueous sodium bicarbonate and dried to give 9-amino-3,3-dimethyl-6-hydroxy-1,2,3,4-tetrahydroacridine (0.77 g).
mp : 260° C (dec.)
IR (Nujol) : 3460, 3380, 3220, 1645, 1600 $cm^{-1}$
NMR (DMSO-$d_6$, δ) : 0.95 (6H, s), 1.59 (2H, t, J=6Hz), 2.47 (2H, t, J=6Hz), 2.55 (2H, s), 6.50 (2H, s), 6.83 (1H, dd, J=2Hz, 9Hz), 6.89 (1H, d, J=2Hz), 7.99 (1H, d, J=2Hz)

**Claims**

1. A compound of the formula :

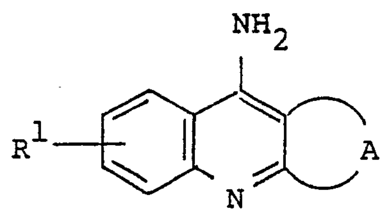

wherein $R^1$ is hydrogen, halogen, hydroxy, lower alkoxy, lower alkyl or mono(or di or tri)halo(lower)alkyl, and

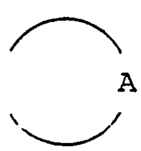 is 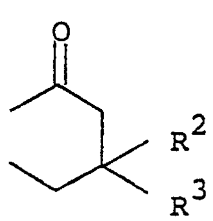, 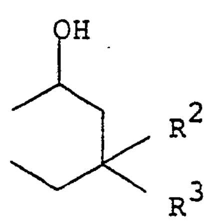, 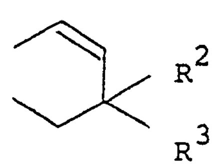,

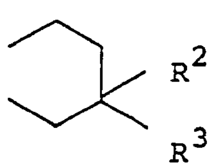, 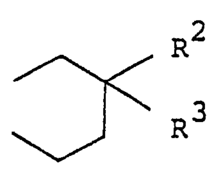 or 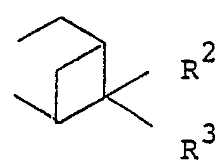

(in which $R^2$ and $R^3$ are each lower alkyl),
and a pharmaceutically acceptable salt thereof.

2. A compound of claim 1,
wherein $R^1$ is hydrogen, halogen, hydroxy, $C_1$-$C_4$ alkoxy, $C_1$-$C_4$ alkyl or mono(or di or tri)halo($C_1$-$C_4$)alkyl, and

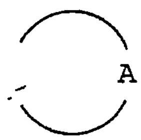 is 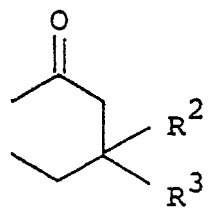, 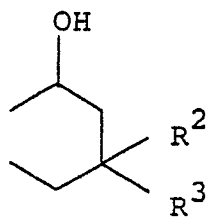, 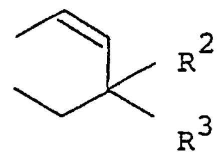,

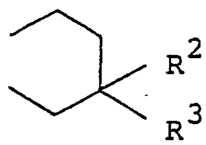, 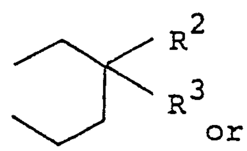 or 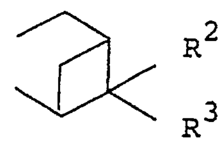

(in which $R^2$ and $R^3$ are each $C_1$-$C_4$ alkyl).

3. A process for preparing a compound of the formula :

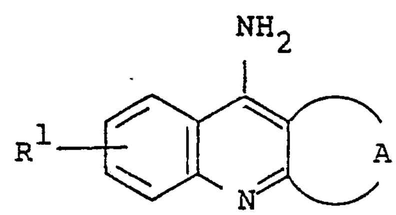

wherein $R^1$ is hydrogen, halogen, hydroxy, lower alkoxy, lower alkyl or mono(or di or tri)halo(lower)alkyl, and

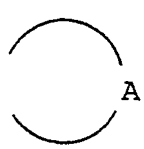 is 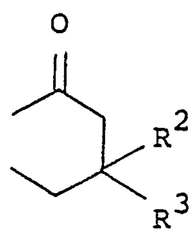, 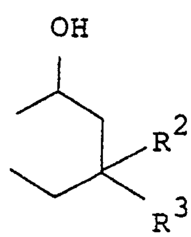, 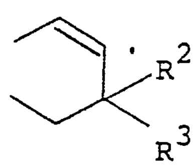,

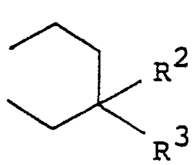, 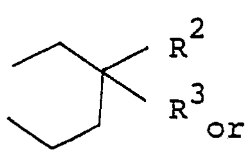 or 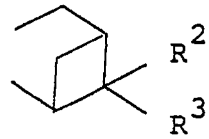

(in which $R^2$ and $R^3$ are each lower alkyl),
or a salt thereof,
which comprises

(1) subjecting a compound of the formula :

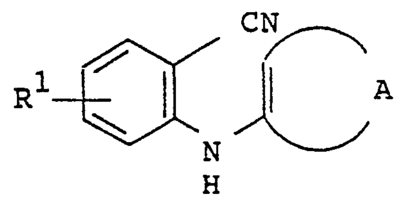

wherein $R^1$ and

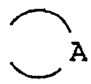

are each as defined above,
or a salt thereof to cyclization reaction to give a compound of the formula :

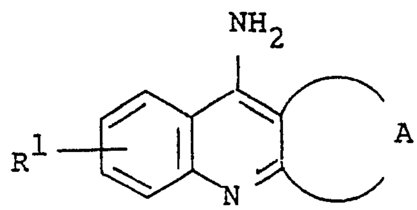

wherein $R^1$ and

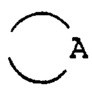

are each as defined above,
or a salt thereof, or

(2) subjecting a compound of the formula :

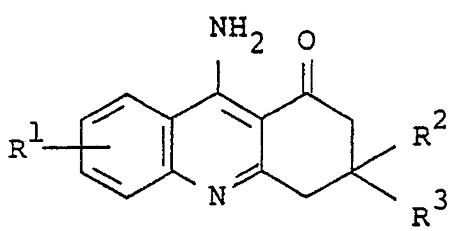

wherein $R^1$, $R^2$ and $R^3$ are each as defined above,
or a salt thereof to reduction reaction to give a compound of the formula :

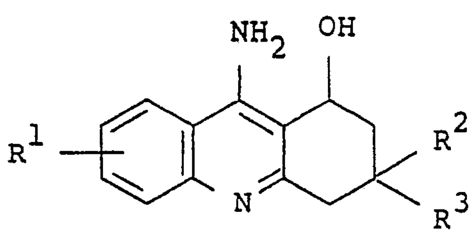

wherein $R^1$, $R^2$ and $R^3$ are each as defined above, or a salt thereof, or

(3) subjecting a compound of the formula :

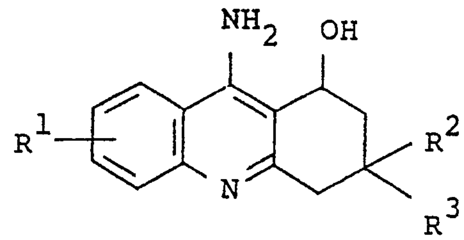

wherein $R^1$, $R^2$ and $R^3$ are each as defined above, or a salt thereof to dehydration reaction to give a compound of the formula :

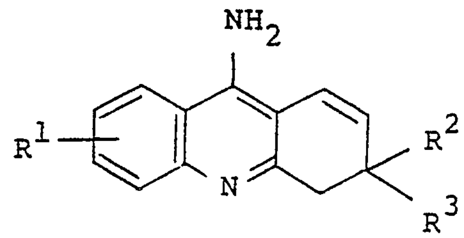

wherein $R^1$, $R^2$ and $R^3$ are each as defined above, or a salt thereof, or

(4) subjecting a compound of the formula :

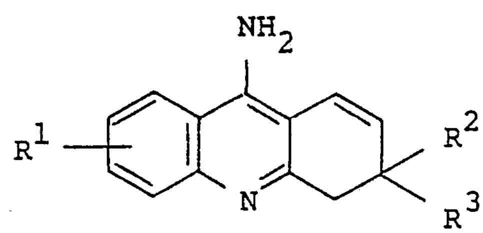

wherein $R^1$, $R^2$ and $R^3$ are each as defined above, or a salt thereof to reduction reaction to give a compound of the formula :

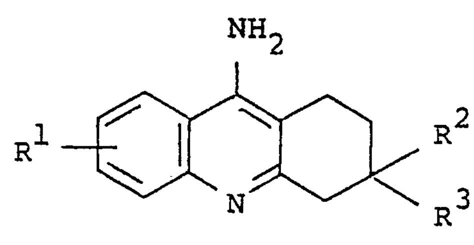

wherein $R^1$, $R^2$ and $R^3$ are each as defined above, or a salt thereof, or

(5) reacting a compound of the formula :

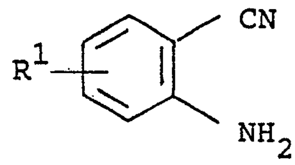

wherein $R^1$ is as defined above, or a salt thereof with a compound of the formula :

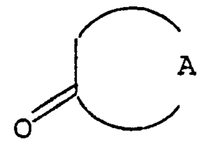

wherein

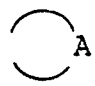

is as defined above, or a salt thereof to give a compound of the formula:

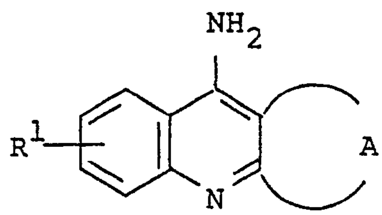

wherein $R^1$ and

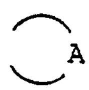

are each as defined above, or a salt thereof, or

(6) subjecting a compound of the formula :

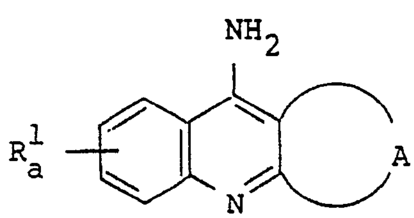

wherein

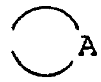

is as defined above,
$R_a^1$ is lower alkoxy,
or a salt thereof to dealkylation reaction to give a compound of the formula :

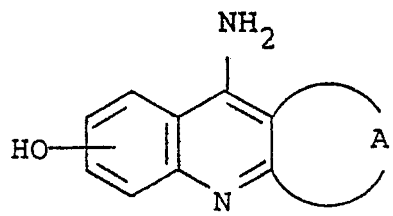

wherein

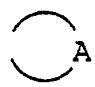

is as defined above,
or a salt thereof.

4. A compound of the formula :

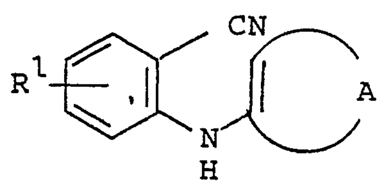

wherein $R^1$ is hydrogen, halogen, hydroxy, lower alkoxy, lower alkyl or mono(or di or tri)halo(lower)alkyl, and

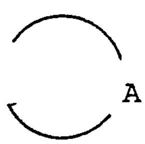 is 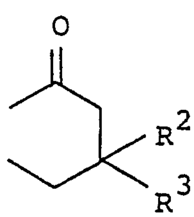 , 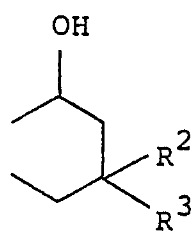 , 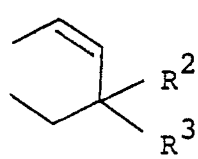 ,

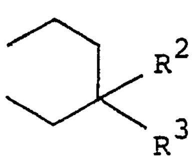 , 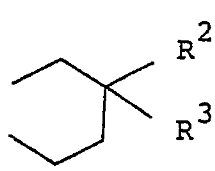 or 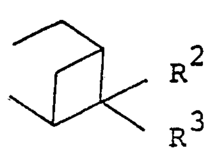

(in which $R^2$ and $R^3$ are each lower alkyl),
or a salt thereof,

5. A process for preparing a compound of the formula :

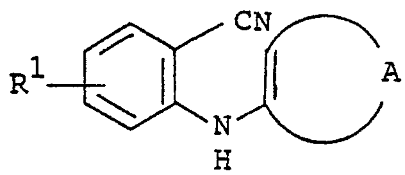

wherein $R^1$ is hydrogen, halogen, hydroxy, lower alkoxy, lower alkyl or mono(or di or tri)halo(lower)alkyl, and

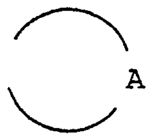 is 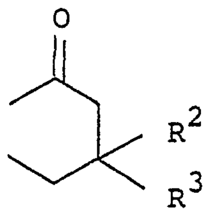, 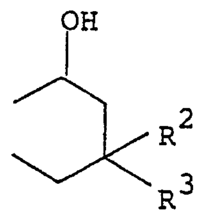, 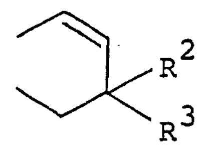,

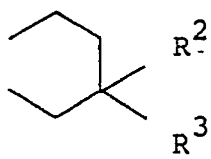, 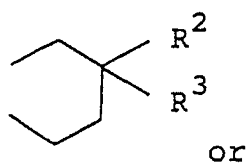 or 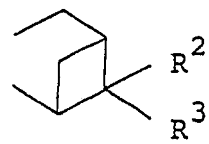

(in which $R^2$ and $R^3$ are each lower alkyl),
or a salt thereof,
which comprises reacting a compound of the formula :

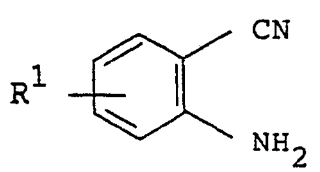

wherein $R^1$ is as defined above,
or a salt thereof with a compound of the formula :

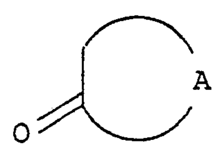

wherein

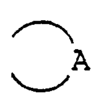

is as defined above,
or a salt thereof.

6. A pharmaceutical composition which comprises, as an active ingredient, a compound of claim 1 or a pharmaceutically acceptable salt thereof in admixture with pharmaceutically acceptable carriers.

7. A compound of claim 1 or a pharmaceutically acceptable salt thereof for use as an acetylcholinesterase inhibitor.

8. Use of a compound of claim 1 or a pharmaceutically acceptable salt thereof for the manufacture of a medicament for use as an acetylcholinesterase inhibitor.